(19) Europäisches Patentamt
European Patent Office
Office européen des brevets

(11) Publication number: **0 012 470**
**B1**

(12) # EUROPEAN PATENT SPECIFICATION

(45) Date of publication of patent specification: **24.11.82**

(51) Int. Cl.³: **C 07 D 209/52**

(21) Application number: **79200713.0**

(22) Date of filing: **03.12.79**

(54) **Isomerisation of pyrrolidine derivatives.**

(30) Priority: **14.12.78 GB 4848978**

(43) Date of publication of application:
**25.06.80 Bulletin 80/13**

(45) Publication of the grant of the patent:
**24.11.82 Bulletin 82/47**

(84) Designated Contracting States:
**BE CH DE FR GB IT NL SE**

(56) References cited:
**FR - A - 2 324 236**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Searle, Robert John Griffith**
**"Highworth" Stockers Hill Rodmersham Green**
**Sittingbourne, Kent (GB)**
Inventor: **Day, Janet Anne**
**3, Palmerstone Walk**
**Sittingbourne, Kent (GB)**

(74) Representative: **Rogers, Roy Charles et al,**
**4 York Road**
**London SE1 7NA (GB)**

**EP 0 012 470 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Isomerisation of pyrrolidine derivatives

This invention relates to a process for the isomerisation of derivatives of 3-azabicyclo-[3.1.0]hexane.

2-Carboxy-3-azabicyclo[3.1.0]hexane and certain derivatives thereof have exhibited extremely useful plant growth regulating and pollen suppressing properties (in this regard, see FR—A—2 324 326). The single isomer cis (L) 2-carboxy-3-azabicyclo[3.1.0]hexane is found in the seeds of the American horse chestnut in small amounts, and much research has been directed to developing a synthetic method of producing this and the other isomers in larger amounts.

A method has now been found by which trans 2-carboxy-3-azabicyclo[3.1.0]hexane and derivatives thereof can be converted into the corresponding cis isomer.

This invention provides a process for the conversion of the trans isomer of an ester of an acid having the formula

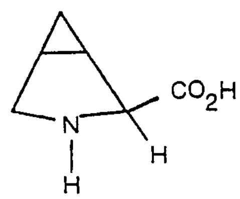

(I)

into the cis isomer of such an ester or the corresponding free acid or a salt thereof, which comprises converting the trans isomer into an ester of an acid having the formula

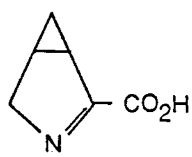

(II)

and subsequently hydrogenating said compound; and, in the event of an ester resulting and an acid or salt being required, converting the resulting ester into the corresponding free acid or a salt thereof.

Various possibilities for isomerism exist, of course, depending upon the nature of substituents on the bicyclic nucleus. However, throughout this specification, a trans isomer should be understood to be a geometric isomer in which the $—CO_2—$ group in the 2-position of the 3-azabicyclohexane ring is trans to the bridging group in the 6-position, and a cis isomer is a geometric isomer in which the $—CO_2—$ group is cis to the bridging group.

It should be noted that for each such geometric isomer, a pair of optical isomers exists, due to the asymmetry of the 2-carbon atom.

The starting trans ester may be either pure trans ester or it may be present in a mixture of cis and trans esters. Thus any mixture of cis and trans esters may be converted into cis compounds, or into a mixture of trans and cis compounds containing an enhanced proportion of cis isomer, by the process of the invention.

If desired, the product obtained directly from the hydrogenation process, if it is an ester, may be converted into the corresponding free acid or a salt thereof. This may be performed by any suitable method, for example hydrolysis under acidic or basic conditions, and if desired subsequent conversion of a resulting free acid into a salt thereof, or conversion of a resulting salt into the corresponding free acid. Hydrogenation of certain esters may produce the free cis acid directly, if the starting ester moiety is one which is cleaved by hydrogenolysis. Thus, if the desired product of the process of the invention is a free acid, a preferred embodiment of the process comprises starting from such an ester, preferably a benzyl ester. Hydrogenolysis of the benzyl ester of an acid having the formula II, leads not only to hydrogenolysis of the C = N double bond giving a cis isomer, but also leads to cleavage of the ester moiety. Thus a free cis acid is obtained directly. The starting ester may if desired be prepared by esterification of the free acid or a salt thereof. Thus the process according to the invention may in effect be used to convert the trans isomer of an acid having the formula I, or a salt thereof, into the cis isomer of such an acid, or a salt or an ester thereof.

Preferably the carbon skeletons I and II are substituted only by hydrogen atoms.

The starting ester may for example be an alkyl, alkenyl, alkynyl, aryl or aralkyl ester in which the ester moiety may be unsubstituted or substituted by one or more of the same or different substituents selected from halogen atoms, alkyl groups and alkoxy groups. Preferably the ester is an alkyl, alkenyl or aralkyl ester in which the ester moiety is unsubstituted. More preferably the ester is an alkyl ester having from 1 to 10, especially 1 to 4 carbon atoms in the alkyl group, or a benzyl ester. For example, the ester may be a methyl, ethyl, isopropyl or benzyl ester. It is of course possible to prepare the cis

isomer of an ester in which the ester moiety is different from the starting ester moiety. For example, esters containing unsaturated groups may produce cis esters by which the ester moiety has been hydrogenated to a saturated group.

Conversion of the starting ester to the ester of the acid II may be carried out directly using an oxidising agent. Manganese dioxide is a suitable reagent, and the oxidation can be performed simply by stirring the ester of the compound of formula II with manganese dioxide in the presence of a suitable solvent, for example a hydrocarbon such as benzene or light petroleum. The reaction is conveniently performed at room temperature.

Alternatively, the conversion may be carried out indirectly for example by chlorinating or brominating an ester of an acid having the formula I to give an ester of an acid whose carbon skeleton includes the unit

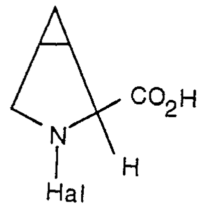

(III)

in which Hal is a chlorine or bromine atom, and subsequently dehydrohalogenating the N-halo compound. The halogenation may be carried out using any suitable halogenating agent, for example N-bromo- or, especially, N-chlorosuccinimide, or an organic or inorganic hypohalite, for example t-butyl hypochlorite or sodium hypochlorite. Sodium hypochlorite may conveniently be used in the form of sodium hydroxide plus chlorine. The halogenation is suitably carried out by admixing the halogenating agent with the starting ester. Any suitable solvent, for example an ether, may be used. The reaction may for example be carried out a temperature in the range of from —10°C to +30°C, the optimum temperature depending on the halogenating agent used. For example, if a hypohalite is used as halogenating agent, the reaction is preferably carried out at a temperature in the range of from —10°C to +5°C, whereas if N-chlorosuccinimide is used as halogenating agent, the reaction is most conveniently carried out at room temperature.

Suitable dehydrohalogenating agents for the dehydrohalogenation step include organic bases and inorganic bases, for example an alkali metal hydroxide or alkoxide. Care should be taken however to ensure that the reaction conditions are such that the ester group is not attacked. For this reason, the base used should be relatively non-nucleophilic; for example, sodium ethoxide is generally preferred to sodium hydroxide. The reaction may be carried out in any suitable polar solvent, for example an ether or an alcohol, and is preferably carried out at a temperature of up to 150°C, preferably at a temperature in the range 0° to 80°C.

The resulting azabicyclohexene derivative may be isolated by any suitable method, or at least part of the reaction mixture containing the azabicyclohexene derivative may be used directly in the subsequent hydrogenation step.

The hydrogenation step of the process according to the invention may for example be carried out by using a hydride transfer agent; preferably however it is carried out using gaseous hydrogen in the presence of a homogeneous or, preferably, heterogeneous catalyst, for example a palladium or platinum catalyst, such as palladium charcoal or, preferably, pre-reduced platinum oxide. The process may be carried out at atmospheric pressure but it is preferably carried out at elevated pressure, for example up to 150 atmospheres gauge.

The hydrogenation reaction is preferably carried out at room temperature. It may however if desired be carried out at elevated temperature, for example up to 100°C.

If the alcohol from which the ester starting material is derived is a liquid, the hydrogenation reaction is preferably carried out using this alcohol as solvent. Thus in one embodiment of the process, an ethyl ester is used as starting material and the hydrogenation is carried out using ethanol as solvent. However any other suitable solvent may be used if desired, for example an ester, for example ethyl acetate, or an aromatic hydrocarbon, for example toluene. If the ester starting material is itself a liquid, the reaction may be carried out without the use of a solvent.

The following Examples illustrate the invention.

## Example 1

(a) N-chlorination of trans 2-ethoxycarbonyl-3-azabicyclo[3.1.0]hexane

Tert.-butyl hypochlorite (3.34 g, 0.03 mol) was added dropwise to a stirred and ice-cooled solution of trans 2-ethoxycarbonyl-3-azabicyclo[3.1.0]hexane (4.65 g, 0.03 mol) in 90 ml dry ethyl ether, under a nitrogen atmosphere and with the exclusion of light. The temperature rose from 0° to 10°C. The solution was stirred for 5 minutes.

(b) Preparation of 2-ethoxycarbonyl-3-azabicyclo[3.1.0]hex-2-ene

Sodium (0.69 g) was dissolved in dry ethanol (90 ml) and the resulting solution was added quickly to the solution of 2-ethoxycarbonyl-3-chloro-3-azabicyclo[3.1.0]hexane obtained in (a) above. An exothermic reaction ensued, and the mixture was stirred and cooled for 30 minutes. A fine white precipitate which could not be filtered was produced. The solvent was evaporated off and the residue was taken up in ether, washed twice with water and dried over sodium sulphate. On evaporation, 4.8 g of 2-ethoxycarbonyl-3-azabicyclo[3.1.0]hex-2-ene were obtained as an oil.

(c) Preparation of cis 2-ethoxycarbonyl-3-azabicyclo[3.1.0]hexane

Adams catalyst (0.1 g) consisting of platinum oxide, was added to 20 ml ethanol and shaken under hydrogen at a pressure of 2 atmospheres gauge for 15 minutes, to reduce it to platimum prior to use. A solution of 2-ethoxycarbonyl-3-azabicyclo[3.1.0]hex-2-ene (1.5 g, 0.01 mol) in diethyl ether (20 ml) was added to the suspension of pre-reduced catalyst, and the mixture was shaken under hydrogen at 2 atmospheres gauge and at room temperature, for five hours. The Mixture was filtered through "Hyflo" (Trademark), a commercial filtering aid, and the solvent was removed by evaporation. 1.3 g of 2-ethoxycarbonyl-3-azabicyclo[3.1.0]hexane as a pale yellow oil which crystallised on standing, were obtained. NMR could detect the presence of no trans isomer.

(d) Preparation of cis 2-carboxy-3-azabicyclo[3.1.0]hexane

2M sodium hydroxide (20 ml) was added to cis 2-ethoxycarbonyl-3-azabicyclo[3.1.0]hexane (1.2 g) and the suspension was stirred at room temperature for 1 hour, after which time the oil had all dissolved. The solution was stirred for a further 4 hours at room temperature and left to stand overnight. It was then passed through a column of Amberlite 410 resin in the $OH^-$ form. 1 l of water and then 1 1 2N hydrochloric acid were used as eluents. The resulting solvent was evaporated and the residue was applied to a column of Amberlite IRA 120 resin in the $H^+$ form, using water and then dilute ammonium hydroxide as eluents. ("AMBERLITE" is a Trade Mark).

1 g of 2-carboxy-3-azabicyclo[3.1.0]hexane was obtained, which was shown by NMR to be almost all in the cis isomeric form.

## Example 2

The procedure of Example 1 was repeated, except that 2-isopropoxycarbonyl-3-azabicyclo-[3.1.0]hexane (92% trans isomer, 8% cis isomer) was used as starting material in step (a); potassium hydroxide dissolved in isopropyl alcohol was used as dehydrohalogenating agent in step (b); and a 5% palladium charcoal catalyst in isopropyl alcohol was used in step (c). The resulting product was 2-isopropoxycarbonyl-3-azabicyclo[3.1.0]hexane which was shown by NMR to contain 85% cis isomer, 15% trans isomer.

## Example 3

The procedure of Example 1 was repeated, except that the procedure of steps (a) and (b) combined were replaced by the following.

Manganese dioxide (2 g) was added to a solution of 2-ethoxycarbonyl-3-azabicyclo[3.1.0]hexane (1 g) in 50 ml of petrol, boiling point 40—60°C. The resulting suspension was stirred at room temperature under a nitrogen atmosphere, and the reaction was monitored by gas-liquid chromatography. After 4 hours, a further 2 g of manganese dioxide was added.

The suspension was left overnight at room temperature, and then filtered and evaporated down. 0.85 g of solid 2-ethoxycarbonyl-3-azabicyclo[3.1.0]hex-2-ene were obtained, corresponding to a yield of 87%.

## Example 4

The procedure of Example 2 was repeated except that steps (a) and (b) were replaced by the following.

A solution of 2-isopropoxycarbonyl-3-azabicyclo[3.1.0]hexane (8.45 g, 0.05 mol) in 100 ml dry diethyl ether, was added dropwise over 10 minutes to a stirred suspension of N-chlorosuccinimide (11.48 g; 0.086 mol) in 200 ml dry diethyl ether, under nitrogen and protected from light. The mixture was stirred at 25—30°C for $2\frac{1}{2}$ hours. The mixture was then washed with brine (3 × 25 ml), dried over sodium sulphate, filtered, and cooled to 0°C. A solution of sodium isopropoxide (prepared by dissolving 1.15 g of sodium in 100 ml dry isopropylalcohol) and 100 ml dry dichloromethane, was added dropwise at 0°C under nitrogen, in the dark. The suspension was stirred at 0°C for 1 hour, evaporated down, and the residue treated with water (100 ml) and diethyl ether (500 ml). The ether solution was then dried over sodium sulphate, and evaporated down. 5.9 g of an orange liquid was obtained. This was distilled under vacuum under a nitrogen atmosphere. 2.7 g of 2-isopropoxycarbonyl-3-azabicyclo-[3.1.0]hex-2-ene were obtained.

## Example 5

(a) Tert.butyl hypochlorite (1.84 g, 0.017 mol) was added dropwise to a solution of the benzyl ester of

2-carboxy-3-azabicyclo[3.1.0]hexane (3.69 g, 0.017 mol), containing 84% of trans isomer in dry diethyl ether (70 ml) at 0 to 5°C under nitrogen. The solution was stirred at 0 to 5°C, in the dark, for 15 minutes, then freshly-distilled triethylamine (5 ml) was added. The suspension was stirred at room temperature for 2 days, and was then evaporated down. The residue was shaken with diethyl ether (500 ml) and water (25 ml), and the ether layer was separated, washed with water (25 ml), dried over sodium sulphate and evaporated down. The residue was purified chromatographically to give 2.3 g of 2-benzyloxycarbonyl-3-azabicyclo[3.1.0]hex-2-ene.

(b) A solution of the unsaturated benzyl ester (1.33 g) in 40 ml absolute ethanol was added to a slurry of 5% palladium charcoal (0.65 g) in 10 ml ethanol, under nitrogen. The mixture was then shaken under hydrogen at room temperature for $1\frac{1}{2}$ hours. The mixture was filtered, and the aqueous ethanolic filtrate was evaporated to give a straw-coloured glassy solid. NMR showed that this solid was 2-carboxy-3-azabicyclo[3.1.0]hexane, with a cis:trans ratio of 93:7.

## Claims

1. A process for the conversion of the trans isomer of an ester of an acid having the formula

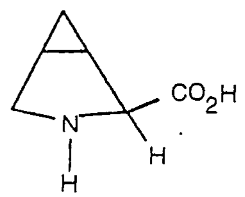

(I)

into the cis isomer of such an ester or the corresponding free acid or a salt thereof, which comprises converting the trans isomer into an ester of an acid having the formula

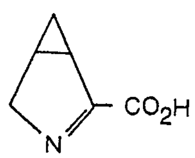

(II)

and subsequently hydrogenating said compound; and in the event of an ester resulting and an acid or salt being required, converting the resulting ester into the corresponding free acid or a salt thereof.

2. A process as claimed in Claim 1, characterised in that the starting ester is converted into the ester II by reaction with an oxidising agent.

3. A process as claimed in Claim 2, characterised in that the oxidising agent is manganese dioxide.

4. A process as claimed in Claim 1, characterised in that the starting ester is converted into the ester II by chlorinating or brominating the starting ester to give an ester of an acid having the formula

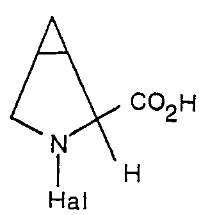

(III)

in which Hal is a chlorine or bromine atom, and subsequently dehydrohalogenating the N-halo compound.

5. A process as claimed in Claim 4, characterised in that the starting ester is chlorinated using N-chlorosuccinimide, t-butyl hypochlorite or sodium hypochlorite.

6. A process as claimed in either Claim 4 or Claim 5, characterised in that the halogenation is carried out at a temperature in the range of from −10°C to +30°C.

7. A process as claimed in any one of Claims 4 to 6, characterised in that the dehydrohalogenation step is carried out using an alkali metal hydroxide or alkoxide.

8. A process as claimed in any one of Claims 4 to 7, characterised in that the dehydrohalogenation step is carried out at a temperature of up to 150°C.

9. A process as claimed in any one of Claims 1 to 8, characterised in that the hydrogenation step is carried out using gaseous hydrogen in the presence of a heterogeneous catalyst.

10. A process as claimed in Claim 9, characterised in that the catalyst comprises palladium or platinum.

11. A process as claimed in any one of Claims 1 to 10, characterised in that the alcohol from which the starting ester is derived is a liquid, and the hydrogenation step is carried out using this alcohol as solvent.

12. A process as claimed in any one of Claims 1 to 11, characterised in that a resulting cis ester is converted into the corresponding free acid or a salt thereof.

13. A process as claimed in any one of Claims 1 to 12, characterised in that the starting ester is an alkyl ester.

14. A process as claimed in any one of Claims 1 to 11, characterised in that the ester of formula II is one in which the ester moiety is cleaved by hydrogenolysis, thus producing the cis isomer of the free acid of formula I.

15. A process as claimed in Claim 14, characterised in that the starting ester is the benzyl ester.

**Revendications**

1. Procédé pour la transformation de l'isomère trans d'un ester d'un acide ayant la formule

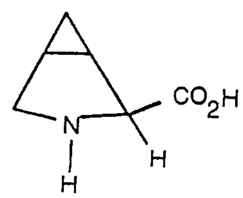

(I)

en l'isomère cis de cet ester ou en l'acide libre correspondant ou en un sel de cet acide, selon lequel on transforme l'isomère trans en un ester d'un acide ayant la formule

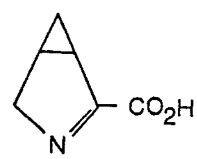

(II)

et ensuite on hydrogène ce composé; et dans le cas où il en résulte un ester et où on désire un acide ou un sel, on transforme l'ester résultant en l'acide libre correspondant ou en un sel de cet acide.

2. Procédé selon la revendication 1, caractérisé en ce que l'ester de départ est transformé en l'ester II par réaction avec un agent oxydant.

3. Procédé selon la revendication 2, caractérisé en ce que l'agent oxydant est du bioxyde de manganèse.

4. Procédé selon la revendication 1, caractérisé en ce que l'ester de départ est transformé en l'ester II par chloration ou bromation de l'ester de départ pour donner un ester d'un acide ayant la formule

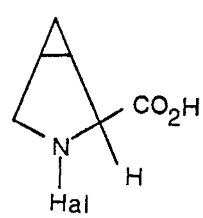

(III)

dans laquelle Hal est un atome de chlore ou de brome, et ensuite déshalogénhydratation du composé N-halogéné.

5. Procédé selon la revendication 4, caractérisé en ce qu'on chlore l'ester de départ en utilisant du N-chlorosuccinimide, de l'hypochlorite de t-butyle ou de l'hypochlorite de sodium.

6. Procédé selon la revendication 4 ou 5, caractérisé en ce que l'halogénation est effectuée à une température comprise entre −10°C et +30°C.

7. Procédé selon l'une quelconque des revendications 4 à 6, caractérisé en ce qu'on conduit l'étape de déshalogénhydratation en utilisant un hydroxyde ou alcoolate de métal alcalin.

8. Procédé selon l'une quelconque des revendications 4 à 7, caractérisé en ce qu'on conduit l'étape de déshalogénhydratation à une température allant jusqu'à 150°C.

9. Procédé selon l'une quelconque des revendications 1 à 8, caractérisé en ce qu'on conduit l'étape d'hydrogenation en utilisant de l'hydrogène gazeux en présence d'un catalyseur hétérogène.

10. Procédé selon la revendication 9, caractérisé en ce que le catalyseur comprend du palladium ou du platine.

11. Procédé selon l'une quelconque des revendications 1 à 10, caractérisé en ce que l'alcool duquel l'ester de départ est dérivé est un liquide, et qu'on conduit l'étape d'hydrogénation en utilisant cet alcool comme solvant.

12. Procédé selon l'une quelconque des revendications 1 à 11, caractérisé en ce qu'un ester cis résultant est transformé en l'acide libre correspondant ou en un sel de cet acide.

13. Procédé selon l'une quelconque des revendications 1 à 12, caractérisé en ce que l'ester de départ est un alcoyl ester.

14. Procédé selon l'une quelconque des revendications 1 à 11, caractérisé en ce que l'ester de formule II est un composé dans lequel la portion est clivée par hydrogénolyse, produisant ainsi l'isomère cis de l'acide libre de formule I.

15. Procédé selon la revendication 14, caractérisé en ce que l'ester de départ est le benzyl ester.

**Patentansprüche**

1. Verfahren zur Umwandlung des trans-Isomeren eines Esters von einer Säure der Formel

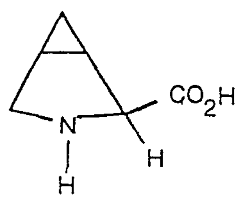

(I)

in das cis-Isomere eines solchen Esters oder der entsprechenden freien Säure oder eines Salzes davon, umfassend die Umwandlung des trans-Isomeren in einen Ester einer Säure der Formel

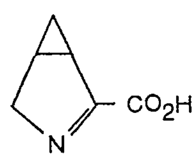

(II)

und anschließende Hydrierung dieser Verbindung und im Falle, daß ein Ester entsteht und eine Säure oder ein Salz erwünscht ist, Umwandlung des entstandenen Esters in die entsprechende freie Säure oder ein Salz davon.

2. Verfahren nach Anspruch 1, dadurch gekennzeichnet, daß der Ausgangsester in den Ester II umgewandelt wird durch Reaktion mit einem Oxidationsmittel.

3. Verfahren nach Anspruch 2, dadurch gekennzeichnet, daß das Oxidationsmittel Mangandioxid ist.

4. Verfahren nach Anspruch 1, dadurch gekennzeichnet, daß der Ausgangsester in den Ester II umgewandelt wird durch Chlorierung oder Bromierung des Ausgangsesters unter Bildung eines Esters einer Säure der Formel

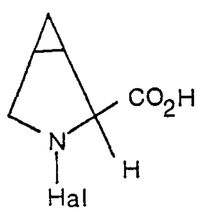

(III)

in der Hal ein Chlor- oder Bromatom bedeutet, und anschließende Dehydrohalogenierung der N-Halogenverbindung.

5. Verfahren nach Anspruch 4, dadurch gekennzeichnet, daß der Ausgangsester mit Hilfe von N-Chlorsuccinimid, tert.-Butylhypochlorit oder natriumhypochlorit chloriert wird.

6. Verfahren nach Anspruch 4 oder 5, dadurch gekennzeichnet, daß die Halogenierung bei einer Temperatur im Bereich von −10 bis +30°C durchgeführt wird.

7. Verfahren nach einem der Ansprüche 4 bis 6, dadurch gekennzeichnet, daß die Dehydrohalogenierung mit Hilfe eines Alkalihydroxids oder -alkoxids durchgeführt wird.

8. Verfahren nach einem der Ansprüche 4 bis 7, dadurch gekennzeichnet, daß die Dehydrohalogenierung bei einer Temperatur von bis zu 150°C durchgeführt wird.

9. Verfahren nach einem der Ansprüche 1 bis 8, dadurch gekennzeichnet, daß die Hydrierung durchgeführt wird unter Verwendung von gasförmigem Wasserstoff in Gegenwart eines heterogenen Katalysators.

10. Verfahren nach Anspruch 9, dadurch gekennzeichnet, daß der Katalysator Palladium oder Platin enthält.

11. Verfahren nach einem der Ansprüche 1 bis 10, dadurch gekennzeichnet, daß der Alkohol, von dem der Ausgangs-Ester abgeleitet ist, flüssig ist und die Hydrierung durchgeführt wird unter Verwendung dieses Alkohols als Lösungsmittel.

12. Verfahren nach einem der Ansprüche 1 bis 11, dadurch gekennzeichnet, daß der erhaltene cis-Ester in die entsprechende freie Säure oder ein Salz davon umgewandelt wird.

13. Verfahren nach einem der Ansprüche 1 bis 12, dadurch gekennzeichnet, daß der Ausgangsester ein Alkylester ist.

14. Verfahren nach einem der Ansprüche 1 bis 11, dadurch gekennzeichnet, daß der Ester der Formel II ein solcher ist, bei dem die Estergruppe durch Hydrogenolyse abgespalten wird unter Bildung des cis-Isomeren der freien Säure der Formel I.

15. Verfahren nach Anspruch 14, dadurch gekennzeichnet, daß der Ausgangsester der Benzylester ist.